(19) Europäisches Patentamt
European Patent Office
Office européen des brevets

(11) **EP 4 578 496 A1**

(12) **EUROPEAN PATENT APPLICATION**

(43) Date of publication:
**02.07.2025 Bulletin 2025/27**

(21) Application number: 24217498.5

(22) Date of filing: **04.12.2024**

(51) International Patent Classification (IPC):
**A61N 5/10** *(2006.01)*     **G21G 4/06** *(2006.01)*

(52) Cooperative Patent Classification (CPC):
**A61N 5/1001; G21G 4/06;** A61N 2005/1024

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.12.2023 TW 112151400**

(71) Applicant: **Platinum Optics Technology Inc.**
**Taoyuan City 33383 (TW)**

(72) Inventors:
• **TSAI, Yu-Yu**
  **33383 Taoyuan City (TW)**
• **WU, Chien-Hsun**
  **33383 Taoyuan City (TW)**
• **CHEN, Yi-Lin**
  **33383 Taoyuan City (TW)**
• **HSIEH, Hsiang-Wei**
  **33383 Taoyuan City (TW)**

(74) Representative: **Epping - Hermann - Fischer**
**Patentanwaltsgesellschaft mbH**
**Schloßschmidstraße 5**
**80639 München (DE)**

(54) **MICROSPHERE AND PREPARATION METHOD THEREOF**

(57) The present disclosure relates to a microsphere having a plurality of pores that have a gradient characteristic gradually decreasing along the direction from the surface to the center of the microsphere, wherein the microsphere includes: a first nuclide, which is distributed in the microsphere and whose concentration in the center of the microsphere is greater than that in the surface of the microsphere; and a second nuclide and gradually decreasing along the direction from the surface to the center of the microsphere, wherein the first and second nuclides are capable of being radioactive after neutron activation to produce β-rays, γ-rays or a combination thereof. The present disclosure also provides methods of preparing a microsphere. The functions of radiotherapy, imaging and tracking can be achieved by introducing the first and second nuclides into the microsphere, and the plurality of pores can increase the surface area of the microsphere to enhance adhesion with a shell.

FIG. 1A

EP 4 578 496 A1

## FIG. 1B

FIG. 1C

# EP 4 578 496 A1

**Description**

**BACKGROUND**

1. Technical Field

The present disclosure relates to a microsphere used in therapeutic applications and its preparation method, specifically to a microsphere that can be utilized in internal radiation therapy and its preparation method.

2. Description of Associated Art

Internal radiation therapy is a form of radiation therapy. Unlike traditional external beam radiation therapy, which delivers high-energy radiation from outside the body to irradiate tumors within, internal radiation therapy involves the precise placement of a radiation source within the treatment site. For example, a radiation source can be placed inside or adjacent to a tumor to treat it. The primary advantage of internal radiation therapy is that the radiation source is positioned very close to the target, allowing for high-dose treatment; additionally, the irradiation affects only a very limited area around the target, thereby significantly reducing the radiation dose received by normal tissues distant from the radiation source. Furthermore, internal radiation therapy does not require large external irradiation equipment, enabling patients to minimize hospital visits and thereby improving the convenience of medical treatment.

However, internal radiation therapy necessitates the placement of a radiation source within the body, making it crucial to control the precise location of the radiation source. The reported yttrium-90 glass microspheres exhibit good stability but are challenging to trace within the body post-administration, leading to potential concerns.

**SUMMARY**

The present disclosure provides a microsphere with a plurality of pores, which exhibit a gradient characteristic that gradually decreases from the surface to the center of the microsphere. The microsphere comprises:

a first nuclide distributed within the microsphere, with a higher concentration at the center than at the surface; and
a second nuclide distributed within the microsphere, with a concentration that gradually decreases from the surface to the center.,
upon neutron activation, both the first and second nuclides are radioactive, emitting $\beta$-rays, $\gamma$-rays or a combination thereof.

The present disclosure also provides a method for preparing a microsphere, comprising:

providing a first raw material containing a first nuclide and a second raw material containing a second nuclide, both in powder form;
mixing the first and second raw materials to form a mixed powder;
heating the mixed powder to obtain an intermediate product;
melting and spherizing the intermediate product to form a molten droplet;
contacting the molten droplet with a cooling medium to form a microsphere, wherein the first nuclide is more concentrated at the center than at the surface, and the second nuclide's concentration decreases from the surface to the center; and
treating the microsphere with a solution to create a plurality of pores, which exhibit a gradient characteristic decreasing from the surface to the center,
upon neutron activation, both the first and second nuclides are radioactive, emitting $\beta$-rays, $\gamma$-rays or a combination thereof.

The present disclosure further provides an alternative method for preparing a microsphere, comprising:

providing a first raw material containing a first nuclide in powder form;
heating the first raw material to obtain an intermediate product;
melting and spherizing the intermediate product to form a molten droplet;
contacting the molten droplet with a cooling medium to obtain a second nuclide in liquid form, allowing the second nuclide to diffuse into the molten droplet or microsphere from the surface, resulting in a higher concentration of the first nuclide at the center and a decreasing concentration of the second nuclide from the surface to the center; and
treating the microsphere with a solution to create a plurality of pores, which exhibit a gradient characteristic decreasing from the surface to the center,
upon neutron activation, both the first and second nuclides are radioactive, emitting β-rays, γ-rays or a combination thereof.

According to the present disclosure, a microsphere is designed to provide functions such as radiation therapy, imaging, and traceability by incorporating a first and a second nuclide. Additionally, the presence of multiple pores increases the surface area and complexity of the microsphere's morphology, facilitating the addition of a shell layer with excellent adhesion. The shell layer can be modified in various ways to enhance functionality, such as increasing hydrophilicity or hydrophobicity, carrying ligands for targeting specific molecules, or incorporating drugs or other reagents.

## BRIEF DESCRIPTION OF THE DRAWINGS

FIGs. 1A-1C are schematic diagrams showing the structure of a microsphere of the present disclosure.
FIG. 2 is a schematic diagram showing the distribution of a second nuclide in the microsphere of the present disclosure.
FIG. 3 shows SEM images of the glass microspheres of Example 1.
FIG. 4 shows SEM images of the glass microspheres of Examples 3-5.
FIG. 5 shows SEM images of the glass microspheres of Example 7.
FIG. 6 shows SEM images of cross-sections of the glass microspheres of Example 7.
FIG. 7 shows SEM images of the glass microspheres of Example 8.
FIG. 8 shows positron emission tomography (PET)/computed tomography (CT) images of the glass microspheres of Example 9 placed in Eppendorf tubes.
FIG. 9 shows PET/CT images of the glass microspheres of Example 9 placed in agar.
FIG. 10 provides the line charts showing the time-residual activity At relationship of the glass microspheres of Example 9 in Eppendorf tubes or in agar.
FIG. 11 provides the line charts showing the weight-residual activity At relationship of the glass microspheres of Example 9 in Eppendorf tubes or in agar.
FIG. 12 provides the line charts showing the time-ln(At/A0) relationship of the glass microspheres of Example 9 in Eppendorf tubes or in agar.
FIG. 13 provides the line chart showing the time-At/A0 relationship of the glass microspheres of Example 10.
FIG. 14 shows upright fluorescence microscope images of the glass microspheres of Example 11.
FIG. 15 shows diameter distributions of the glass microspheres of Example 11.
FIG. 16 is the line chart showing the time-radiochemical purity of the glass microspheres of Example 12.
FIG. 17 shows PET/CT images of the rats of Example 13, showing the distribution and residual activity At of the glass microspheres in the rats.
FIG. 18 provides the line charts showing the time-residual activity At relationship and time-ln(At/A0) relationship of the glass microspheres of Example 13 in rats.

## DETAILED DESCRIPTION OF THE EMBODIMENTS

The embodiments of the present invention will be illustrated by specific examples hereafter. One having ordinary skill in the technical field can easily understand the advantages and effects of the present invention after reading the disclosure of this specification.

Note, the structures, proportions, dimensions, etc. shown in the figures attached to the present specification are only for the purpose of assisting one having ordinary skill in the technical field to understand and read, and are not intended to be limiting conditions of the present disclosure, and therefore do not have technical substantive significance. Moreover, any of modifications in structure changes in proportional relationships, adjustments in size, changes or adjustments in relative relationships, should be included within the scope of the disclosure of the present specification, without affecting the efficacy and objectives that can be achieved by the present specification.

Terms such as "first", "second", "upper", and "lower" recited in the specification are used for clear description, the change or adjustment of their relative relationship without substantial alteration of the technical contents are also considered within the implementation scope of the present invention. Furthermore, all ranges and values recited in the present invention are inclusive and combinable. The present invention discloses various ranges, any value or point falling into the ranges described herein is also encompassed by the present invention, and any value or point falling into the ranges described herein can be used as the lower or upper limit to derive a subrange.

Firstly, the first aspect of the present disclosure is a microsphere having a plurality of pores, which exhibit a gradient characteristic that gradually decreases from the surface to the center of the microsphere. The microsphere comprises:

a first nuclide, which is distributed within the microsphere and has a higher concentration at the center than at the surface; and
a second nuclide, which is distributed within the microsphere and has a concentration that gradually decreases from the surface to the center,
upon neutron activation, both the first and second nuclides become radioactive, emitting $\beta$-rays, $\gamma$-rays or a combination thereof.

In an embodiment, the gradient characteristic comprises diameters of the pores, the distribution of the pores, or a combination thereof. Specifically, the definition of "decreases gradually along a direction from the surface to the center of the microsphere" can refer to the diameter or distribution of the pores tending toward being less and less along a direction from the surface to the center of the microsphere. For example, comparing 3 regions randomly selected from the surface to the center of the microsphere, the microsphere has a gradient characteristic of pores decreasing gradually along a direction from the surface to the center of the microsphere if the diameters or distributions in these 3 regions meet the conditions described above. For example, the pore diameter in the microsphere surface region is larger than the pore diameter in the region between the microsphere surface and the microsphere center, and the pore diameter in the region between the microsphere surface and the microsphere center is further larger than the pore diameter in the microsphere center region.

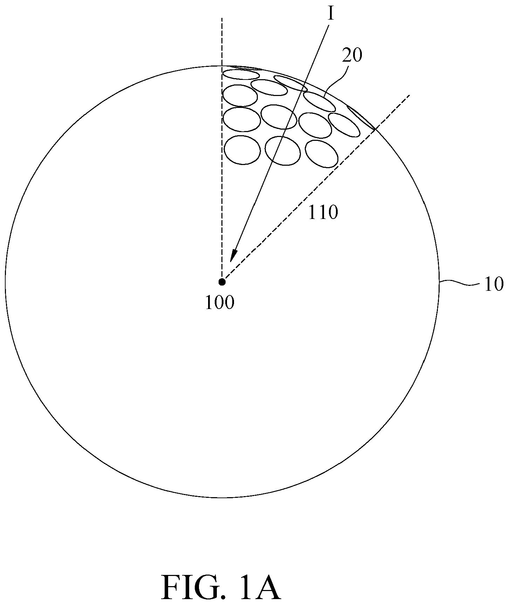

In an embodiment, the plurality of pores of the microsphere of the present disclosure may have a distribution decreased along a direction from the surface to the center of the microsphere. This embodiment can be referenced to FIG. 1A, a microsphere 10 has a plurality of pores 20. There are more pores distributed in the surface region of the microsphere but fewer pores distributed closer to a center 100 of the microsphere, i.e., the pores distribution decreases gradually along the direction I from the surface to the center 100 of the microsphere. In another embodiment, the microsphere of the present disclosure does not have pores distributed throughout them. Referring to FIG. 1A again, there are no pores in the region close to the center 100, and thus it can be considered that the microsphere 10 has a region with a pore gradient characteristic 110 and the pores 20 are only distributed in the region with a pore gradient characteristic 110. The distribution of pores can also be defined as the proportion of the area of all pores relative to the total surface area of the microsphere at a given depth. The higher the proportion, the greater the distribution of pores.

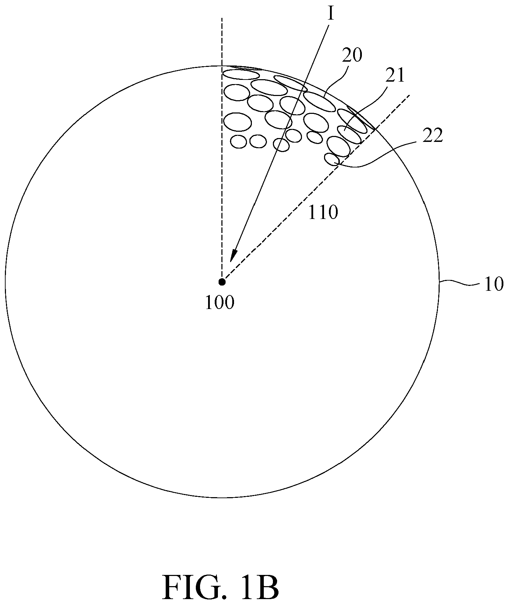

In another embodiment, the plurality of pores of the microsphere of the present disclosure may have a pore diameter decreased along a direction from the surface to the center of the microsphere. This embodiment can be referenced to FIG. 1B, a microsphere 10 has a plurality of pores 20, 21, 22, among which the pore 20 has a larger diameter and is located at or closer to the surface of the microsphere, the pore 22 has a smaller diameter and is closer to the center 100 of the microsphere, and the pore 21 has a diameter and distribution between those of the pore 20 and the pore 22, exhibiting a pore diameter decreased gradually along the direction I from the surface to the center 100 of the microsphere. In another embodiment, similarly, the microsphere of the present disclosure does not have a pores distribution throughout them. Again, referring to FIG. 1B, there are no pores in the region close to the center 100, thus it can be considered that the microsphere 10 has a region with a pore gradient characteristic 110, and the pores 20 are only distributed in the region with a pore gradient characteristic 110.

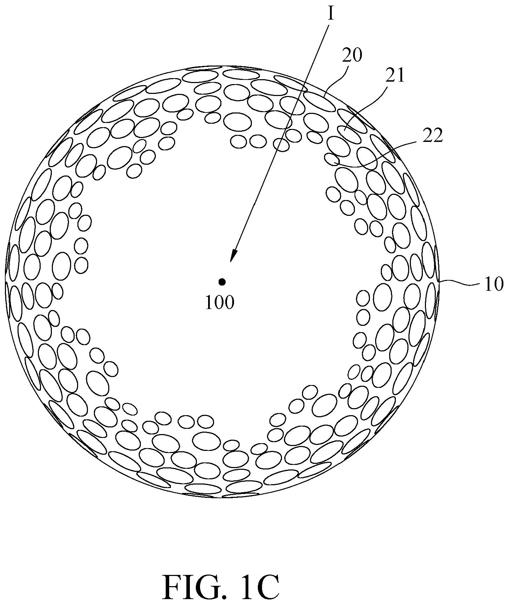

In another embodiment, the plurality of pores of the microsphere of the present disclosure may have a pore diameter and a pore distribution decreased along a direction from the surface to the center of the microsphere. This embodiment can be referenced to FIG. 1C which is the combination of the two embodiments of FIG. 1A and FIG. 1B, and the same content thus is not repeated here.

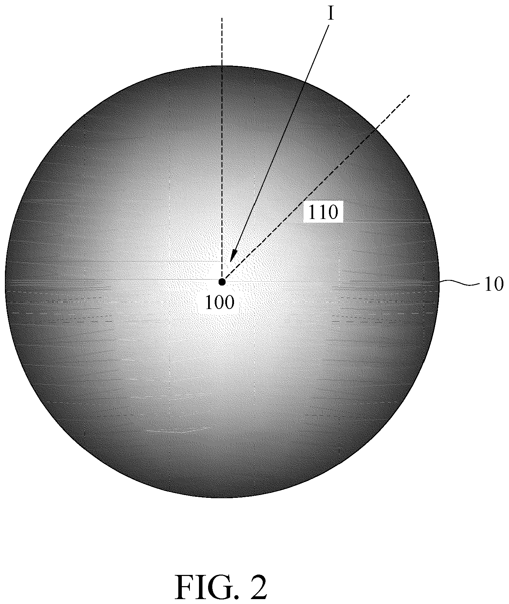

In addition, the distribution of the second nuclide decreasing gradually from the surface to the center of the microsphere can be referred to FIG. 2, the colored portion of the microsphere 10 represents the distribution of the second nuclide which decreases gradually along the direction I from the surface to the center 100 of the microsphere.

In an embodiment, the pores are free from penetrating the microsphere.

In an embodiment, the plurality of pores comprise macropores and micropores, wherein the macropores have diameters of 10-30 microns ($\mu$m), 10-25 $\mu$m, 10-20 $\mu$m, 15-30 $\mu$m, or 20-30 $\mu$m, e.g., 10, 15, 20, 25, or 30 $\mu$m; and the micropores have diameters of 0.1-10 $\mu$m, 0.1-5 $\mu$m, 0.5-10 $\mu$m, or 0.5-5 $\mu$m, e.g., 0.1, 0.5, 1, 5, or 10 $\mu$m. This embodiment can be referenced to FIG. 1C, in which the pore 20 having a larger diameter is as a macropore and the pore 22 having a smaller diameter is as a micropore. In another embodiment, the plurality of pores comprise macropores, mesopores, and micropores, wherein the macropores have diameters of 20-30 $\mu$m, 15-25 $\mu$m, or 20-25 $\mu$m, e.g., 15, 20, 25, or 30 $\mu$m; the mesopores have diameters of 5-20 $\mu$m, 5-15 $\mu$m, 5-10 $\mu$m, or 10-20 $\mu$m, e.g., 5, 10, 15, or 20 $\mu$m; and the micropores have diameters of 0.1-5 $\mu$m, or 0.5-5 $\mu$m, 0.1-1 $\mu$m, or 0.1-10 $\mu$m, e.g., 0.1, 0.5, 1, 5, or 10 $\mu$m. This embodiment can be referenced to FIG. 1C, in which the pore 20 having a larger diameter is as a macropore, the pore 22 having a smaller diameter is as a micropore, and the pore 21 having a diameter between those of the pore 20 and the pore 22 is as a mesopore. In an embodiment, some of the pores are interconnected with each other while others are not, for example, the macropores are not interconnected with each other but are interconnected with some micropores.

In an embodiment, the microsphere is a glass microsphere.

The first nuclide and the second nuclide are not limited in the present disclosure, as long as they can be radioactive after neutron activation to produce β-rays, γ-rays or a combination thereof.

In an embodiment, the first nuclide is capable of providing a β (beta) radioisotope and may be derived from a first raw material. The first nuclide, for example, is selected from yttrium, aluminum, silicon, or a combination thereof. In an embodiment, the first nuclide comprises yttrium, and may also comprise others, for example, but not limited to, aluminum, silicon, or a combination thereof. In an embodiment, the first raw material for forming the first nuclide may be in the form of powder, for example, but not limited to an oxide, for example, but not limited to, yttrium oxide, aluminum oxide, and silicon oxide.

In one embodiment, the second nuclide is capable of providing a γ (gamma) radioisotope and may be derived from a second raw material. The second nuclide may comprise at least one element selected from the group consisting of: potassium, barium, molybdenum, tellurium, indium, antimony, gallium, zinc, zirconium, palladium, rhodium, tantalum, tungsten, iridium, platinum, niobium, technetium, strontium, titanium, vanadium, phosphorus, calcium, sodium, rhenium, scandium, lanthanum, cerium, praseodymium, neodymium, promethium, samarium, europium, gadolinium, terbium, dysprosium, holmium, erbium, thulium, ytterbium, lutetium, copper, gold, silver, iron, tin, cobalt, nickel, manganese, aluminum, carbon, boron, iodine, actinium-225, antimony-127, arsenic-74, barium-140, bismuth-210, bismuth-213, californium-246, calcium-46, calcium-47, carbon-11, carbon-14, cesium-131, cesium-137, chromium-51, cobalt-57, cobalt-58, cobalt-60, dysprosium-165, dysprosium-166, erbium-169, erbium-109, fluorine-18, gallium-67, gallium-68, gold-198, holmium-166, hydrogen-3, indium-111, indium-113m, iodine-123, iodine-125, iodine-131, iridium-192, iridium-194, iron-59, iron-82, krypton-81m, lanthanum-140, lutetium-177, molybdenum-99, nitrogen-13, oxygen-15, palladium-103, phosphorus-32, radon-222, radium-224, radium-223, rhenium-186, rhenium-188, rhodium-82, samarium-153, selenium-75, sodium-22, sodium-24, strontium-89, strontium-90, technetium-99m, thallium-201, xenon-127, xenon-133, cerium-137, actinium-225, zirconium-89, terbium-149, astatine-211, thorium-227, thorium-201, bismuth-212, bismuth-213, copper-64, ytterbium-169, ytterbium-175, lead-212, potassium-42, rubidium-82, titanium-45, scandium-44, and yttrium-90. In an embodiment, the second raw material of the second nuclide may be in the form of powder, for example, but not limited to, an oxide, an oxide hydrate containing crystal water, a hydroxide, etc., for example, but not limited to, yttrium oxide, iron oxide, calcium oxide, aluminum oxide, copper oxide, etc. In another embodiment, the second raw material may be in the form of liquid, for example, but not limited to, copper sulfate containing crystal water, an aqueous solution of copper sulfate, an aqueous solution of iron chloride, an aqueous solution of calcium chloride, an aqueous solution of calcium hydroxide, calcium carbonate containing crystal water, an aqueous solution of aluminum sulfate, an aqueous solution of yttrium acetate, an aqueous solution of phosphoric acid, etc.

The nuclide employed in the present disclosure can be used for radiation therapy when generating a β-ray after neutron activation. It can allow equipment sensing γ-ray to capture the position and the signal of the nuclide when generating a γ-ray after neutron activation. Furthermore, it can be utilized in the applications described above simultaneously when generating both β-ray and γ-ray after neutron activation. Thus, by administrating the microsphere of the present disclosure to a subject, not only can the β-ray generated by the microsphere be utilized to perform radiation therapy on the target tissue, but the distribution and metabolism of the microsphere in the subject can also be observed by capturing the generated γ-ray through imaging equipment (such as a γ-ray camera, a positron tomography scanner, etc.). The integrated application of imaging equipment and a computer can display images and perform calculations and analysis to give more information.

In another aspect, the nuclide contained in the microsphere of the present disclosure is located in a framework. This configuration prevents the nuclide from leaching out easily, thereby avoiding impairment of non-target tissues, unlike other carriers where a nuclide or drug is loaded into pores.

In an embodiment, the microsphere further comprises a shell layer coated on the surface of the microsphere and filled into the plurality of pores.

The microsphere of the present disclosure has an increased surface area and a complex surface morphology due to the plurality of pores, thus a shell layer with excellent adhesion to the microsphere can be easily added on the surface. The shell layer may be subjected to various modifications, such as making it more hydrophilic or hydrophobic, carrying a ligand capable of targeting a specific molecule, or carrying a drug or other reagents, etc., to greatly improve its functionality.

In an embodiment, the shell layer comprises a material selected from an organic material, an inorganic material, or a combination thereof. In a further embodiment, the organic material is selected from polyvinylpyrrolidone, polyvinyl alcohol, carboxymethylcellulose, methylcellulose, hydroxypropylmethylcellulose, hydroxypropylcellulose, polyethylene glycol, Arabic gum, polylactic acid, polylactic acid-glycolic acid, or a combination thereof. In a further embodiment, the inorganic material is selected from phosphate, sulfate, chloride, nitrate, telluride, tellurate, iodide, iodate, xenate, tungstate, rhenate platinate, aurichloride, mercurate, plumbate, bismuthate, astatate, uranate, polonide, osmate, antimonate, stannate, stannide, technetate, molybdate, niobate, bromate, bromide, selenate, selenide, arsenate, zincate, cuprate, cobaltate, ferrate, nickelate, manganate, chromate, vanadate, titanate, chlorate, sulfide, fluorophosphate, fluorosilicate, silicate, aluminate, fluoride, oxide, peroxide, superoxide, cyanate, carbonate, or borate.

In an embodiment, the microsphere has a diameter of 2-1000 microns ($\mu$m). In another embodiment, the microsphere has a diameter of 2-500 $\mu$m, 5-100 $\mu$m, or 5-50 $\mu$m, e.g., 2, 3, 4, 5, 6, 7, 9, 10, 15, 20, 25, 30, 35, 37, 40, 45, 50, 60, 70, 80, 90, 100, 200, 300, 400, 500, 600, 700, 800, 900, or 1000 $\mu$m.

In an embodiment, the first nuclide has an etching resistance higher than that of the second nuclide.

The second aspect of the present disclosure provides a method for manufacturing a microsphere, comprising:

providing a first raw material of a first nuclide and a second raw material of a second nuclide, both in powder form;
mixing the first raw material and the second raw material to form a mixed powder;
heating the mixed powder to obtain an intermediate product;
melting and spherizing the intermediate product to form a molten droplet;
contacting the molten droplet with a cooling medium to form a microsphere, wherein the first nuclide is more concentrated at the center than at the surface, and the second nuclide's concentration decreases from the surface to the center;
treating the microsphere with a solution to create a plurality of pores, which exhibit a gradient characteristic decreasing from the surface to the center of the microsphere,
upon neutron activation, both the first and second nuclides become radioactive, emitting $\beta$-rays, $\gamma$-rays or a combination thereof.

The third aspect of the present disclosure provides a method for preparing a microsphere, comprising:

providing a first raw material of a first nuclide, wherein the first raw material is in powder form;
heating the first raw material to obtain an intermediate product;
melting and spherizing the intermediate product to form a molten droplet;
contacting the molten droplet with a cooling medium to form a microsphere, wherein the cooling medium is a second raw material of a second nuclide in liquid form, allowing the second nuclide to enter the molten droplet or the microsphere from the surface and to be distributed therein or precipitate on the surface, resulting in that the first nuclide is more concentrated at the center of the microsphere than at the surface and the second nuclide's concentration decreases from the surface to the center of the microsphere; and
treating the microsphere with a solution to create a plurality of pores, which exhibit a gradient characteristic decreasing from the surface to the center of the microsphere,
upon neutron activation, both the first and second nuclides become radioactive, emitting $\beta$-rays, $\gamma$-rays or a combination thereof.

In an embodiment, the first and the second raw material may be glass raw material powders. In an embodiment, the first raw material of the first nuclide (glass raw material powder) and/or the second raw material of the second nuclide (glass raw material powder) are used together with other glass raw material powder as a glass raw material powder mixture (i.e., a batch material). The glass raw material powder mixture forms glass after mixing uniformly, melting, and quenching. The formed glass may further be pulverized to give glass powder. For example, a glass raw material powder mixture is placed in a crucible (e.g., a platinum crucible), and the crucible is placed in an electric furnace to melt the glass raw material powder mixture (for a period depending on the components of the powder mixture, generally for 2-8 hours), during which the mixture is optionally stirred to increase the chemical homogeneity; then the crucible is removed and the molten material is allowed to contact with a cooling medium, for example, poured onto a cold steel plate or into cold water, and at this point, the molten material is quenched to form glass. The glass may be pulverized during formation, eliminating the need for a subsequent glass pulverizing step. Finally, the glass is pulverized (e.g., crushing, mechanical grinding, grinding in a ball mill) to produce glass powder.

In an embodiment, the step of heating the first raw material (or mixed powder of the first and the second raw materials) in powder form to obtain an intermediate, the step of melting and spherizing the intermediate, and the step of contacting the molten droplet with a cooling medium may, for example, utilize a flame spraying. For example, the powder is fed into a feeder and then forced air-fed into a pipeline which directs the powder into a gas flame, and at that point, the powder is heated by the gas flame and melts into a molten liquid state and is simultaneously sprayed and flying by the gas flame. During this period, the molten droplet forms a sphere under the influence of multiple factors such as rotation, surface tension, and gravity. Then, the spherical molten droplet contacts a cooling medium and is quenched to form glass.

Upon contacting with a cooling medium as describe above, a rotating spherical molten droplet will draw in a part of the cooling medium; on the other hand, the components in the cooling medium will also diffuse and invade from the surface during the quenching of the spherical molten droplet to form glass. Therefore, in the case that the cooling medium is the second raw material of the second nuclide, i.e., the second raw material is a liquid, the second nuclide will be introduced into the microsphere due to the phenomenon described above and has a distribution decreased gradually from the surface to the center of the microsphere, as shown in FIG. 2.

In the step of contacting the microsphere with a treatment solution to form a plurality of pores, in an embodiment, the treatment solution is used for performing an etching treatment, and in general, the etching treatment starts from the surface of the microsphere. Particularly, in the present disclosure, the microsphere shows excellent resistance to the treatment solution, making the treatment solution only focus on etching the position where the second nuclide located. Therefore, in the case that the second nuclide has a distribution decreased gradually along a direction from the surface to the center of the microsphere as described above, the etching has a degree weakened gradually along the direction from the surface to the center of the microsphere, resulting in a plurality of pores having a gradient characteristic decreased gradually along the direction from the surface to the center of the microsphere. In a further embodiment, the gradient characteristic comprises diameters of the pores, the distribution of the pores, or a combination thereof, as shown in FIGs. 1A-1C.

In an embodiment, the treatment solution comprises an etching agent. In a further embodiment, the etching agent is a combination of one of an acid and a base with an oxidant, the acid is at least one selected from the group consisting of citric acid, lactic acid, oxalic acid, acetic acid, permanganic acid, p-toluenesulfonic acid, phosphoric acid, aqua regia, hydrofluoric acid, hydrochloric acid, hydrobromic acid, hydroiodic acid, nitric acid, sulfuric acid, perchloric acid, chloric acid ($HClO_3$), bromic acid ($HBrO_3$), perbomic acid ($HBrO_4$), iodic acid ($HIO_3$), periodic acid ($HIO_4$), metaperiodic acid ($HIO_4$), selenic acid ($H_2SeO_4$), fluorosilicic acid ($H_2SiF_6$), chlorioplumbic acid ($H_2PbCl_6$), ferric acid ($H_2FeO_4$), fluoroboric acid ($HBF_4$), fluorosulfonic acid ($HSO_3F$), cyanic acid ($HOCN$), thiocyanic acid ($HSCN$), 2,4,6-trinitrophenol ($HC_6H_2N3O_7$), 2,4,6-trinitrobenzoic acid ($HC_7H2N_3O_8$), trifluoroacetic acid ($CF_3COOH$), trichloroacetic acid ($CCl_3COOH$), methane-sulfonic acid ($CH_3SO_3H$), benzenesulfonic acid ($C_6H_5SO_3H$), mercaptocyclohexanesulfonic acid ($C_6H_{10}(SH)SO_3H$), 2-chloroethane thiol ($CH_3CHClSH$), fluoroantimonic acid ($HSbF_6$), magaic acid ($SbF_6SO_3H$), perfluorinated sulfonic acid resin (Nafion-H), chlorofluoroaluminic acid ($HAlCl_3F$), carbonboronoic acid ($H[CHB_{11}Cl_{11}]$), and $FeCl_3 \cdot HClO_4 \cdot SiO_2 \cdot n\text{-}H_2O$; the base is at least one selected from the group consisting of sodium hydroxide, potassium hydroxide, calcium hydroxide, ammonia, lithium hydroxide, rubidium hydroxide, cesium hydroxide, francium hydroxide, strontium hydroxide, barium hydroxide, radium hydroxide, thallous hydroxide, silver diamminohydroxide, choline, quaternary ammonium base, butyl lithium, lithium diisopropylamide, benzyl lithium, Grignard reagent, alkyl copper lithium, sodium methoxide, sodium ethoxide, potassium etyoxide, and sodium tert-butoxide; and the oxidant is at least one selected from the group consisting of an amphoteric compound, hydrogen peroxide, permanganate, hypochlorite, chromate, dichromate, and chromium trioxide.

In a further embodiment, the treatment solution comprises nitric acid and hydrogen peroxide, or the treatment solution comprises sodium hydroxide and hydrogen peroxide. In a further embodiment, the treatment solution is 1-37 wt% of nitric acid (such as 1N) and 1-25 wt% of hydrogen peroxide (such as 25 wt%), or the treatment solution is 0.1-10 N of sodium hydroxide (e.g., 0.1N, 0.5N, 1N, 2N, 5N, 10N) and 1-25 wt% of hydrogen peroxide (such as 25 wt%).

In an embodiment, the step of contacting the microsphere with the treatment solution to form a plurality of pores is performed by optionally heating, e.g., performing an etching treatment at a temperature range of 25°C-100°C, e.g., 25, 30, 35, 40, 45, 50, 55, 60, 65, 70, 75, 80, 95, 100°C. In an embodiment, the step of contacting the microsphere with the treatment solution to form a plurality of pores is performed for 10 minutes (mins) to 3 hours (hrs), e.g., 10 mins, 20 mins, 30 mins, 1 hr, 1.5 hrs, 2 hrs, or 3 hrs.

In an embodiment, the pores are free from penetrating the microsphere, as described in the first aspect.

In an embodiment, the plurality of pores comprise macropores and micropores, wherein the macropores have diameters of 10-30 $\mu$m, 10-25 $\mu$m, 10-20 $\mu$m, 15-30 $\mu$m, or 25-30 $\mu$m, e.g., 10, 15, 20, 20, or 30 $\mu$m; and the micropores have diameters of 0.1-10 $\mu$m, 0.1-5 $\mu$m, 0.5-10 $\mu$m, or 0.5-5 $\mu$m, e.g., 0.1, 0.5, 1, 5, or 10 $\mu$m, as described in the first aspect.

In an embodiment, the microsphere further comprises a shell layer coated on the surface of the microsphere.

In an embodiment, the shell layer comprises a material selected from an organic material, an inorganic material, or a combination thereof. In a further embodiment, the organic material is selected from polyvinylpyrrolidone, polyvinyl alcohol, carboxymethylcellulose, methylcellulose, hydroxypropylmethylcellulose, hydroxypropylcellulose, polyethylene glycol, Arabic gum, polylactic acid, polylactic acid-glycolic acid, or a combination thereof. In a further embodiment, the inorganic material is selected from phosphate, sulfate, chloride, nitrate, or borate.

The present disclosure further illustrates the details more specifically through the following examples. However, the interpretation of the present disclosure shouldn't be considered as limiting that of the following examples.

**EXAMPLES**

Example 1

35 g of $Y_2O_3$ powder, 35 g of $Al_2O_3$ powder, 20 g of $SiO_2$ powder, and 10 g of CuO powder were weighed, mixed uniformly and charged in a crucible, and the crucible was placed in an electric furnace heated at 1600°C to melt the powder mixture. The direct addition of copper was assisted to lower the melting temperature of the bulk glass block by about 100°C. During this time, stirring was performed when melting begun to improve the chemical homogeneity. The crucible was taken out after completion of melting and stirring, and the molten material was poured onto a steel plate to quench and form glass which was pulverized into pieces at the same time. Then, the glass pieces were to about 100 mesh by using a mortar and pestle and were further ground by using a mechanical mortar and pestle or a ball miller until they passed through a sieve of 400 mesh (37 $\mu$m in diameter), to obtain glass powder.

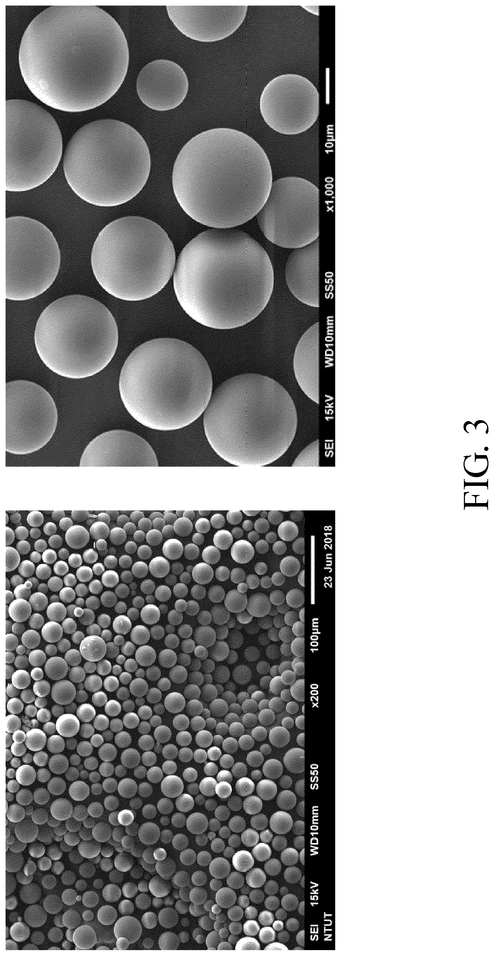

Then, the glass powder was placed in a forced pneumatic feeder over an acetylene/oxygen burner, and through a forced pneumatic delivery, the glass powder entered a pipeline which directed the glass powder into the flame of the acetylene/oxygen burner at a rate set up in the range of 5-25 g/hr. The glass powder melted due to the high temperature of the flame and formed spherical molten droplets during rotation and flight, and the spherical molten droplets contacted with deionized water as a cooling medium to give the glass microspheres of the first aspect. The microspheres having diameters falling into the range of 5-30 $\mu$m were obtained through the sieving, and the microspheres after the sieving described above were measured through a scanning electron microscope (SEM) for the size and morphology, as shown in FIG. 3, for the subsequent experiments. In addition, three microspheres were picked from those after sieving as the samples for energy dispersive X-ray spectra (EDS) analysis, and the results were shown in Table 1 which showed the atom percent of each sample.

Table 1

| | C | O | Al | Si | Cu | Y | Total |
|---|---|---|---|---|---|---|---|
| Sample 1 | | 41.26 | 8.74 | 16.92 | 3.52 | 29.56 | 100.00 |
| Sample 2 | 7.29 | 42.46 | 8.06 | 14.48 | 3.81 | 23.90 | 100.00 |
| Sample 3 | 9.06 | 40.73 | 8.08 | 14.26 | 3.62 | 24.25 | 100.00 |

Example 2

35 g of $Y_2O_3$ powder, 35 g of $Al_2O_3$ powder, and 20 g of $SiO_2$ powder were weighed, mixed uniformly and charged in a crucible, and the crucible was placed in an electric furnace heated at 1700°C to melt the powder mixture. During this time, stirring was performed when melting begun to improve the chemical homogeneity. The crucible was taken out after completion of melting and stirring, and the molten material was poured onto a steel plate to quench and form glass which was pulverized into pieces at the same time. Then, the glass pieces were to about 100 mesh by using a mortar and pestle and were further ground by using a mechanical mortar and pestle or a ball miller until they passed through a sieve of 400 mesh (37 $\mu$m in diameter), to obtain glass powder.

Then, the glass powder was placed in a forced pneumatic feeder over an acetylene/oxygen burner, and through a forced pneumatic delivery, the glass powder entered a pipeline which directed the glass powder into the flame of the acetylene/oxygen burner at a rate set up in the range of 5-25 g/hr. The glass powder melted due to the high temperature of the flame and formed spherical molten droplets during rotation and flight, and the spherical molten droplets contacted with a $CuSO_4 \cdot 5H_2O$ solution (in a supersaturated state with the concentration of 10M) as a cooling medium to give the glass microspheres of the first aspect. The glass microspheres having diameters falling into the range of 5-30 $\mu$m were obtained through sieving for the subsequent experiments.

Example 3

The microspheres of the third aspect were obtained according to the preparation method of Example 2, except that the powder mixture was altered to be 35 g of $Y_2O_3$ powder, 35 g of $Al_2O_3$ powder, 20 g of $SiO_2$ powder, and 10 g of CuO powder.

Example 4

The preparation method of Example 2 was performed, except that the $CuSO_4 \cdot 5H_2O$ solution was altered to one with the concentration of 1M (unsaturated).

Example 5

The preparation method of Example 2 was performed, except that the $CuSO_4 \cdot 5H_2O$ solution was altered to one with the concentration of 3M (supersaturated).

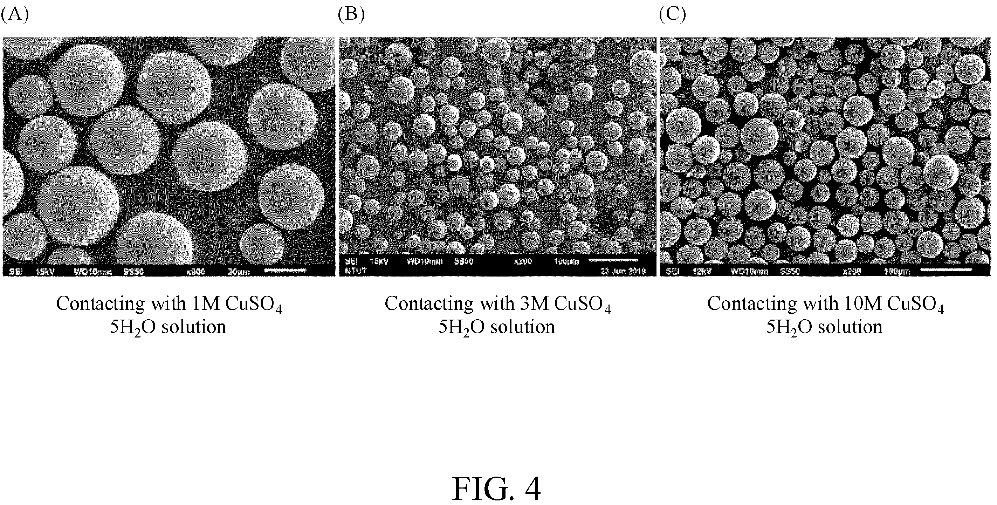

The products of Examples 3-5 were observed under a scanning electron microscope (SEM), and the results were shown in FIG. 4 in which (A)-(C) represented Examples 4, 5 and 2, respectively. According to FIG. 4, no substance precipitation was observed on the surface of the glass microspheres as the glass microspheres contacted with an unsaturated $CuSO_4 \cdot 5H_2O$ solution, but some substances precipitated on the surface of the glass microspheres were observed as the glass microspheres contacted with a supersaturated $CuSO_4 \cdot 5H_2O$ solution.

Example 6

For the glass microspheres of Example 2, five microspheres were randomly taken as the samples for EDS analysis. The results were shown in Table 2 which showed the atom percent of each sample. According to Table 2, the substances precipitated on the surface of the glass microspheres were confirmed to contain copper.

Table 2

|  | O | Al | Si | Cu | Y | Total |
|---|---|---|---|---|---|---|
| Sample 1 | 47.84 | 14.87 | 14.26 | 10.63 | 12.40 | 100.00 |
| Sample 2 | 48.67 | 14.69 | 14.16 | 10.05 | 12.43 | 100.00 |
| Sample 3 | 49.69 | 14.02 | 13.24 | 10.93 | 12.12 | 100.00 |
| Sample 4 | 46.24 | 15.77 | 15.07 | 9.22 | 13.70 | 100.00 |
| Sample 5 | 51.21 | 28.25 | 13.41 | 2.54 | 4.59 | 100.00 |
| Average | 48.73 | 17.52 | 14.03 | 8.67 | 11.05 |  |
| Standard Deviation | 1.87 | 6.03 | 0.74 | 3.49 | 3.66 |  |

In addition, the glass microspheres of Example 2 then contacted with a treatment solution containing 1N nitric acid and 25 wt% of hydrogen peroxide, and such an etching treatment was performed at 75°C for 1 hour. Five microspheres were taken randomly as the samples for EDS analysis. The results were shown in Table 3 which showed the atom percent of each sample. According to Table 3, copper precipitated on the surface of the glass microspheres was still observed with the average concentration of copper reduced from 8.67 to 6.80, suggesting that a portion of copper had been etched off.

Table 3

|  | O | Al | Si | Cu | Y | Total |
|---|---|---|---|---|---|---|
| Sample 1 | 44.14 | 9.26 | 16.21 | 5.92 | 24.47 | 100.00 |
| Sample 2 | 43.80 | 8.91 | 14.30 | 6.48 | 26.51 | 100.00 |
| Sample 3 | 41.03 | 8.78 | 14.36 | 6.87 | 28.96 | 100.00 |
| Sample 4 | 40.87 | 8.91 | 14.84 | 6.62 | 28.76 | 100.00 |
| Sample 5 | 41.78 | 8.41 | 14.00 | 8.10 | 27.71 | 100.00 |
| Average | 42.32 | 8.85 | 14.74 | 6.80 | 27.28 |  |
| Standard Deviation | 1.55 | 0.31 | 0.87 | 0.81 | 1.85 |  |

Example 7

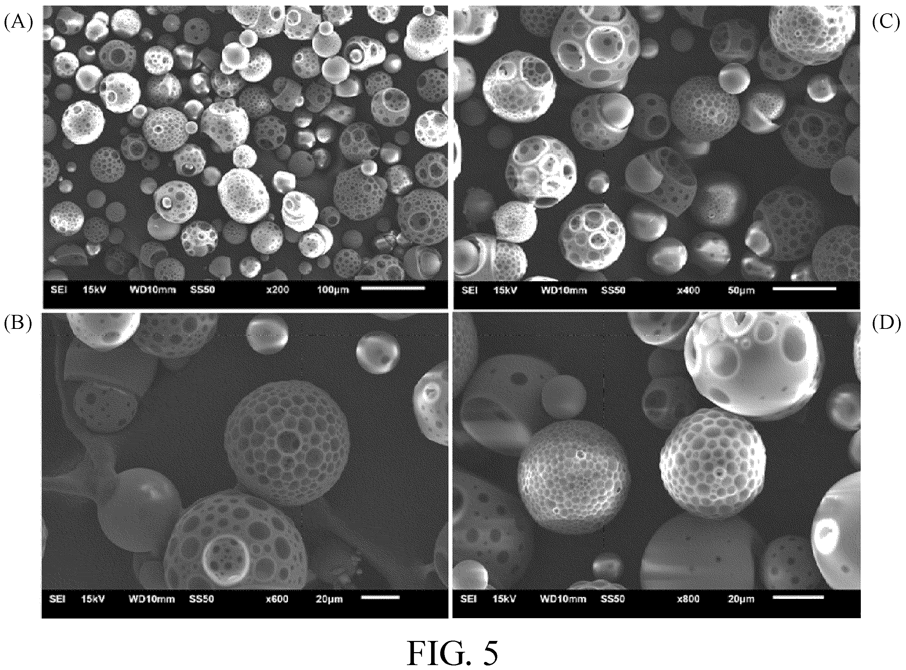

The glass microspheres of Example 2 contacted with a treatment solution containing 1N nitric acid and 25 wt% of hydrogen peroxide, and such an etching treatment was performed at 75°C for 24 hours. The results of the SEM images thereof were shown in (A), (B), (C), and (D) of FIG. 5 which showed that the glass microspheres were etched to have a plurality of pores. And, for the glass microspheres after the etching treatment of this Example, two microspheres were randomly taken as the samples for EDS analysis, and the results were shown in Tables 4 and 5 which showed the atom percent and weight percent of each sample.

Table 4

| Sample 1 | O | Al | Si | Cu | Y | Total |
|---|---|---|---|---|---|---|
| wt% | 45.19 | 8.64 | 16.29 | 2.84 | 27.04 | 100.00 |
| Atom% | 69.33 | 7.86 | 14.24 | 1.10 | 7.47 | 100.00 |
|  |  |  |  |  |  |  |
| Standard | $SiO_2$ | $Al_2O_3$ | $SiO_2$ | Cu | Y |  |

Table 5

| Sample 2 | O | Al | Si | Cu | Y | Total |
|---|---|---|---|---|---|---|
| wt% | 46.04 | 8.35 | 15.99 | 2.48 | 27.14 | 100.00 |
| Atom% | 70.18 | 7.55 | 13.88 | 0.95 | 7.44 | 100.00 |
|  |  |  |  |  |  |  |
| Standard | $SiO_2$ | $Al_2O_3$ | $SiO_2$ | Cu | Y |  |

Next, 100 mg of glass microspheres after the etching treatment were taken. A double-sided carbon adhesive tape was tiled on an electro-conductive substrate. The glass microspheres were tiled on the double-sided carbon adhesive tape after the double-sided carbon adhesive tape was dried, and purged with a gas gun. Glass microspheres capable of sufficiently adhering to the carbon adhesive would be attached to the substrate, and the glass microspheres after the etching treatment of this Example were still attached to the substrate sufficiently after purging with a gas gun.

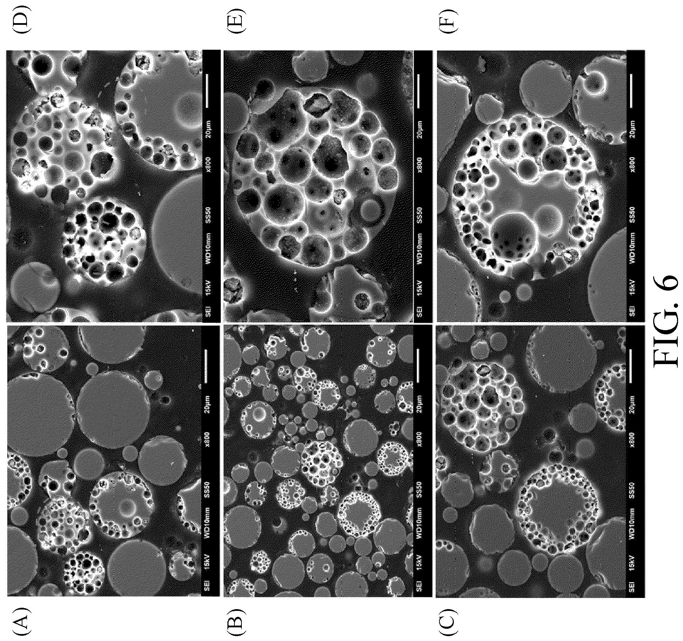

To observe the sections of the glass microspheres after the etching treatment of Example 7, the glass microspheres after the etching treatment were further subjected to treatments including embedding, grinding, polishing, washing, and electro-conductive layer plating, and the SEM images of the sections of the glass microspheres were shown in (A)-(F) of FIG. 6. The treatment steps described above were as follows:

1. Cold embedding

100 mg of glass microspheres were washed (can refer to Step 4) and placed in a mold and a mixture of resin and hardening agent was also poured into the mold. Hardening was performed under normal pressure and temperature. Then, the embedded specimen was taken out and was cut into a suitable size.

2. Grinding

The embedded specimen was ground using a SiC sandpaper with coarse grinding and fine grinding were performed according to the following numbers: 240→400→600→800→1200→2400→4000.

3. Polishing

The surface of the specimen was polished to a mirror surface (no scratches when observed under an optical microscope). A diamond suspension was used as a polishing solution, in which diamond abrasive particles can quickly remove materials and flatten the surface of the specimen.

4. Washing

The specimen was soaked in alcohol and deionized water and subjected to ultrasonication to remove the residues left on the specimen during the grinding and polishing steps, thereby avoiding the interference of the residues on analysis results.

5. Electro-conductive Layer Plating

The non-electro-conductive glass microspheres and the resin into which the glass microspheres were embedded needed to be plated with an electro-conductive layer to prevent the concentration of charged electron beams. The electro-conductive layer was, e.g., a continuous thin film of Au.

Example 8

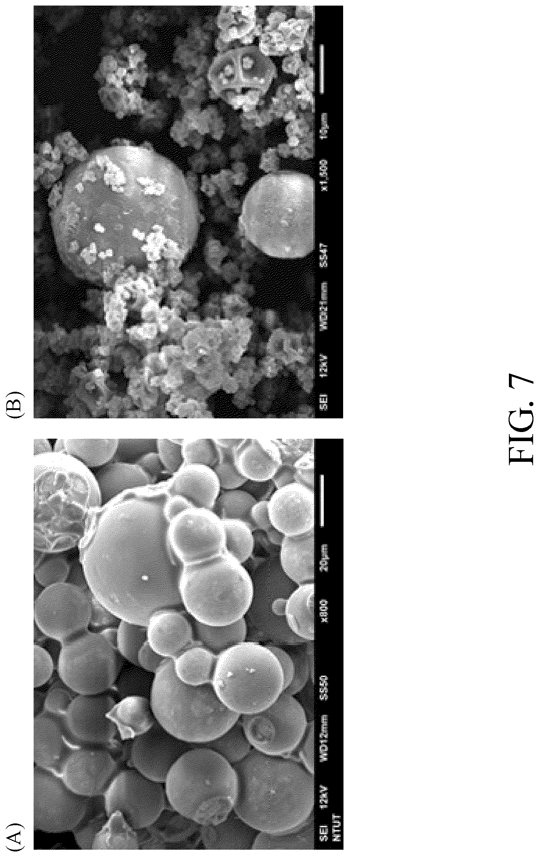

The glass microspheres having a plurality of pores after the etching treatment for 24 hours of Example 7 were taken out. Then, the glass microspheres having a plurality of pores were subjected to spray granulation to be coated with the materials listed in Table 6 below. The glass microspheres were wet and mixed uniformly with 0.1-10 wt% of the material solution at a ratio of 1:1, and then the mixture was subjected to a spray drier for coating, wherein the flow rate was set at 357 L/h, the outlet temperature was between 180-245°C. All groups could form a shell layer on the surface of the microsphere. As shown in FIG. 7, the SEM images of the glass microspheres coated with PVP (A) and the glass microspheres coated with CMC (B) showed that an obvious shell layer had been formed on the surface of the glass microsphere.

Table 6

| Shell layer material | Forming the shell |
|---|---|
| Polyvinylpyrrolidone (PVP) | Yes |
| Polyvinyl alcohol (PVA) | Yes |
| Carboxymethylcellulose (CMC) | Yes |
| Polyethylenen glycol (PEG) 6000 | Yes |
| Methylcellulose (MC) | Yes |
| Hydroxypropylmethylcellulose (HPMC) | Yes |
| Hydroxypropylcellulose (HPC) | Yes |
| Arabic gum | Yes |
| Polylactic acid-glycolic acid (PLA/PGA) | Yes |
| $Ca_3(PO_4)_2$ | Yes |

Example 9

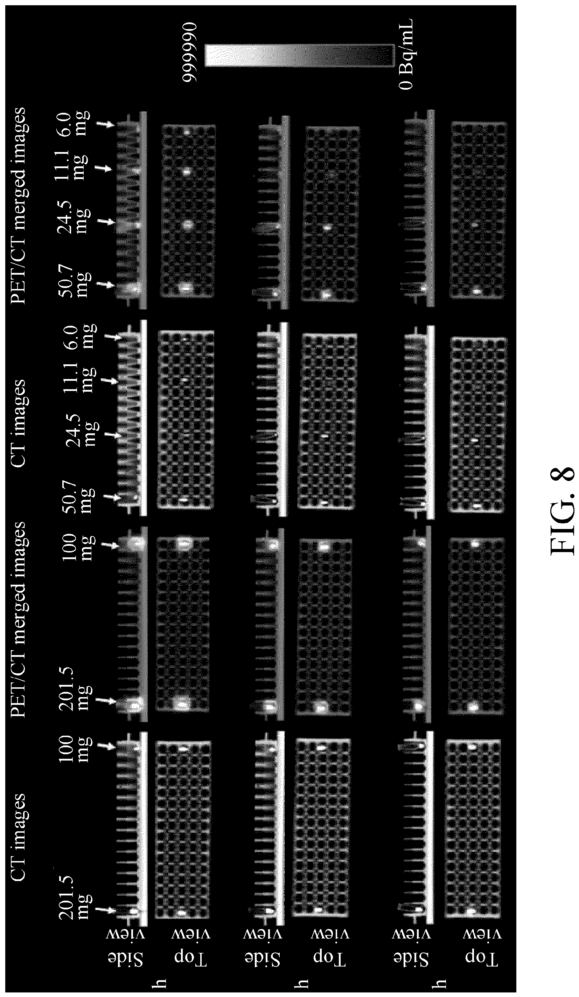

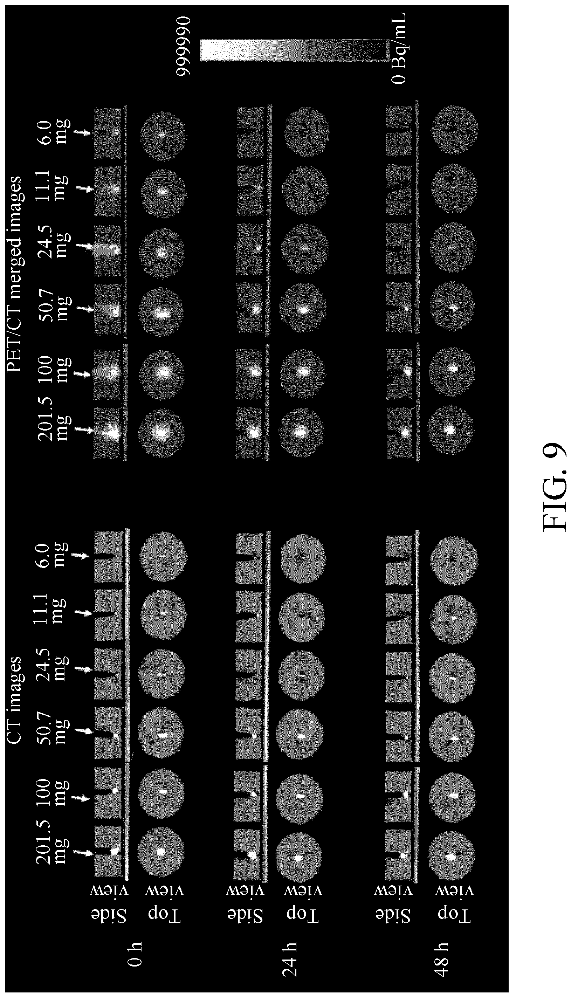

To confirm whether the positron signals generated by the decay of yttrium-90 and copper-64 in the glass microspheres can be detected by using positron emission tomography (PET)/computed tomography (CT), the glass microspheres of Example 3 were allowed to contact with a treatment solution containing 1N nitric acid and 25 wt% of hydrogen peroxide, and an etching treatment was performed at 75°C for 1 hr to form glass microspheres having a plurality of pores. 5 mg, 10 mg, 25 mg, 50 mg, 100 mg, and 200 mg of glass microspheres were taken out (each for two group samples) and placed into 0.5 mL Eppendorf tubes, respectively. After being irradiated with neutron radiation for 6 hours, the glass microspheres were allowed to decay overnight. One group sample was measured for the radioactive activity every 6 hours with a dose corrector and was subjected to positron emission tomography/computed tomography (PET/CT), and the other group sample was placed in a columnar agar and was scanned in the same manner. FIG. 8 showed the PET/CT images of samples placed in Eppendorf tubes, and FIG. 9 showed the PET/CT images of samples placed in the agar.

The results in FIG. 8 and FIG. 9 showed that whether in Eppendorf tubes or in agar, from the images acquired at different times, completely overlaid CT and PET images of the glass microspheres can be seen, with that only the positron signals in PET images were attenuated over time. And, the intensities of the positron signal at each time were correlated with the weight of the glass microsphere.

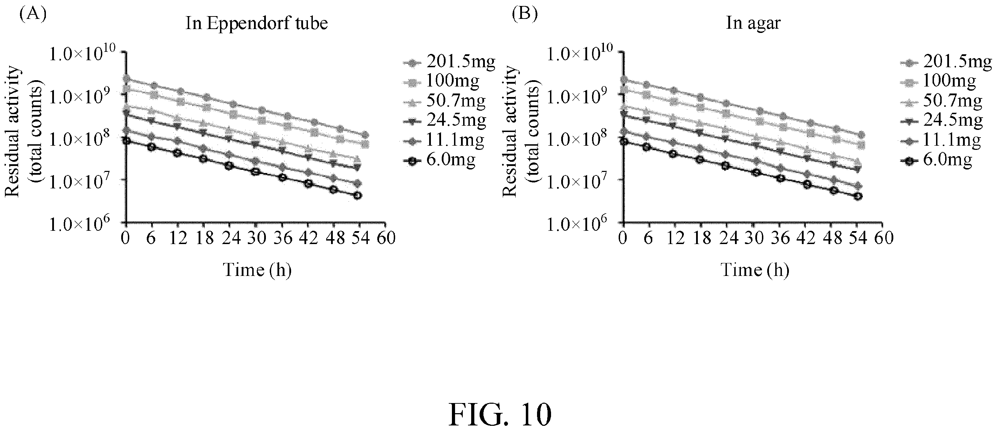

Then, regions of interest (ROIs) in FIG. 8 and FIG. 9 were picked with an image analysis software, and the photon signal intensities in the ROIs were calculated to perform a quantification, thereby giving a half-life. (A) and (B) of FIG. 10 depicted the time-residual activity At relationship charts (TAC), and the results showed that the positron signal decayed over time and in a linear relationship, being consistent with the decay function of a radioactive material.

Calculation:

$$A_t = A_0 \times e^{-\lambda t} \quad \text{[Equation 1]}$$

wherein At is the residual activity of a radioactive material after decay for time t;
$A_0$ is the activity of the radioactive material at the initiation time;
t is the time past; and
$\lambda$ is the decay constant.

A linear function can be obtained by taking the natural logarithm of both sides of the equal sign in Equation 1:

$$\ln A_t = -\lambda t + \ln A_0 \quad \text{[Equation 2]}$$

$$\ln A = 2.303 \times \log A$$

$$2.303 \log A_t = -\lambda t + 2.303 \log A_0 \quad \text{[Equation 3]}.$$

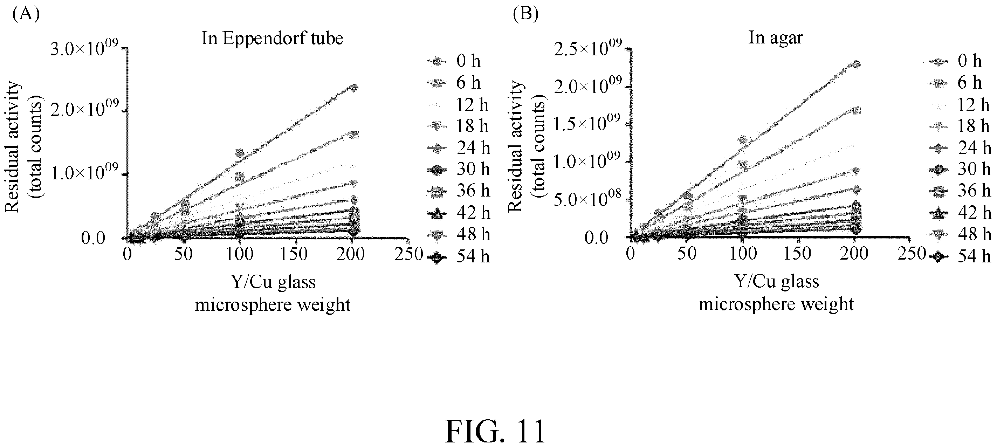

In addition, the yttrium-90/copper-64 dual nuclides glass microspheres having different weights were placed in Eppendorf tubes and agar and subjected to PET/CT imaging at different times. The photon signal intensities in ROIs of the images were calculated. FIGs. 11(A) and (B) depicted the weight-residual activity At relationship charts (TAC), respectively, wherein the linear correlation coefficient ($R^2$) in each chart was larger than 0.99, exhibiting the highly linear correlation between the positron signal intensity and the glass microsphere weight at each time

In this Example, the decay function of the radioactive material described above was derived using the following equation which divides both sides of the equal sign in Equation 1 by $A_0$ and then takes the natural logarithm to obtain:

$$\ln \frac{A_t}{A_0} = -\lambda t \quad \text{[Equation 4]},$$

while $t = t_{1/2}$, $A = \frac{1}{2} A_0$ ($t_{1/2}$ represented the half-life of the radioactive nuclide):

$$\lambda = \frac{0.693}{t_{1/2}} \quad \text{[Equation 5]}.$$

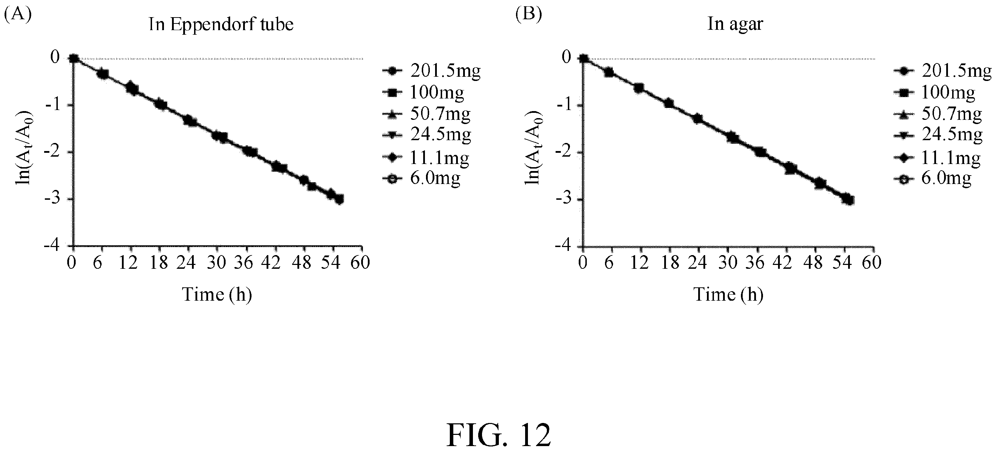

The time-$\ln(A_t/A_0)$ relationship charts according to Equation 4 as shown in FIG. 12, wherein (A) and (B) showed the half-lives of the radioactive materials contained in the glass microspheres placed in Eppendorf tubes and in agar, respectively. Each curve in the figure was subjected to linear regression with the GraphPad Prism software to obtain the slope of each curve, i.e., the decay constant $\lambda$ in Equation 4, and the values were then substituted into Equation 5 to calculate the half-lives of the radioactive materials, which were listed in Table 7.

Table 7

| Actual weight of glass microsphere (mg) | | 6.0 | 11.1 | 24.5 | 50.7 | 100 | 201.5 | AVG$\pm$SD |
|---|---|---|---|---|---|---|---|---|
| Placed in Eppendorf tube rack | Slope | -0.055 | -0.054 | -0.055 | -0.054 | -0.054 | -0.055 | -0.055$\pm$0.000 |
| | Half-life (h) | 12.72 | 12.78 | 12.62 | 12.80 | 12.78 | 12.61 | 12.72$\pm$0.08 |
| Placed in agar | Slope | -0.054 | -0.054 | -0.055 | -0.055 | -0.054 | -0.055 | -0.055$\pm$0.000 |
| | Half-life (h) | 12.75 | 12.83 | 12.69 | 12.58 | 12.73 | 12.70 | 12.71$\pm$0.08 |

According to these results, each group of glass microspheres, whether in Eppendorf tubes or in agar, had an average half-life of 12.7 hours which was the same as that of copper-64, therefore, it could be confirmed that the positron decay signal of copper-64 could be detected when the yttrium-90/copper-64 glass microspheres were subjected to PET/CT. Without being bound by theory, a PET imaging instrument had coupling circuits and an energy window selection and thus only detected paired 511 keV energy photons generated by positron-electron annihilation, with other non-positron decay photon signals filtered out. In addition, although yttrium-90 had a small amount of positron decay, the proportion was very low, about $3.18 \times 10^{-5}$, having a minor influence.

Example 10

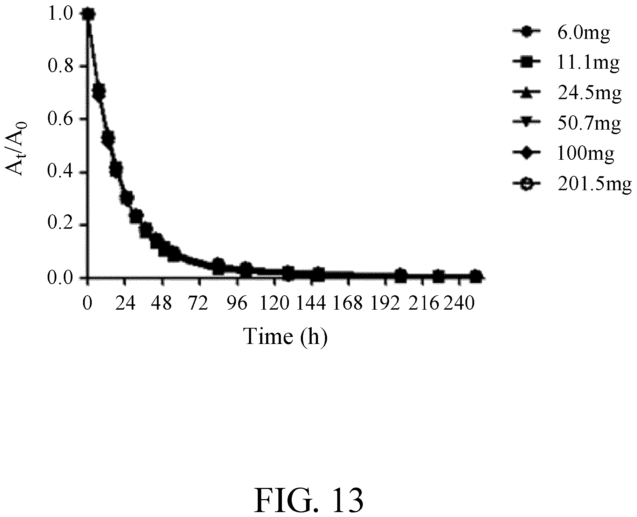

To further confirm that in addition to the copper-64 radioactive nuclide, yttrium-90 radioactive nuclide was also contained in the yttrium-90/copper-64 dual nuclides glass microspheres, in this Example, the activity of the yttrium-90/copper-64 dual nuclides glass microsphere was read with a dose corrector which could detect γ-ray and β-ray with a higher energy. Firstly, the detection period was set to 250 hours (about 4 times of the half-life of yttrium-90), and the readouts of the dose corrector were plotted into a time-$A_t/A_0$ relationship chart, as shown in FIG. 13. The curves were subjected to 2-phase curve regression analysis with the GraphPad Prism software to obtain the half-lives of the two radioactive materials: 66.3 hours and 12.7 hours, which were close to or the same as the known yttrium-90 half-life (64.1 hours) and copper-64 half-life (12.7 hours). Thus, it could be confirmed that the glass microspheres after neutron activation mainly contained two radioactive nuclides yttrium-90 and copper-64.

Example 11

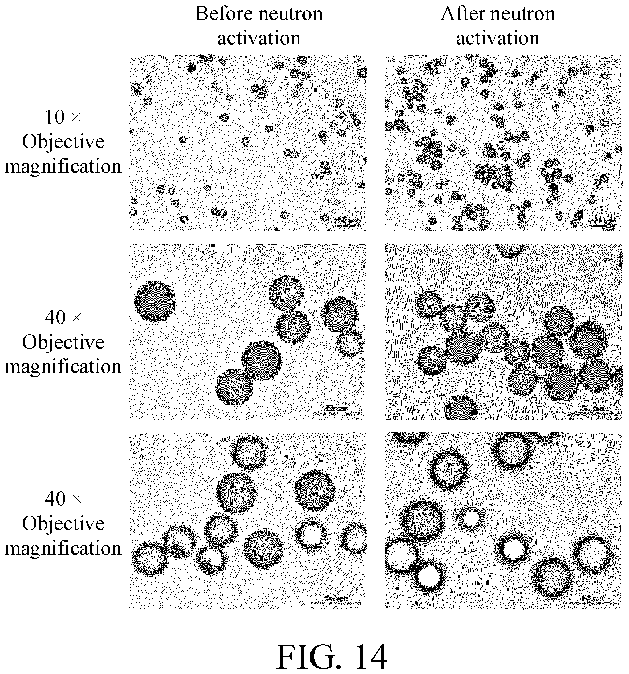

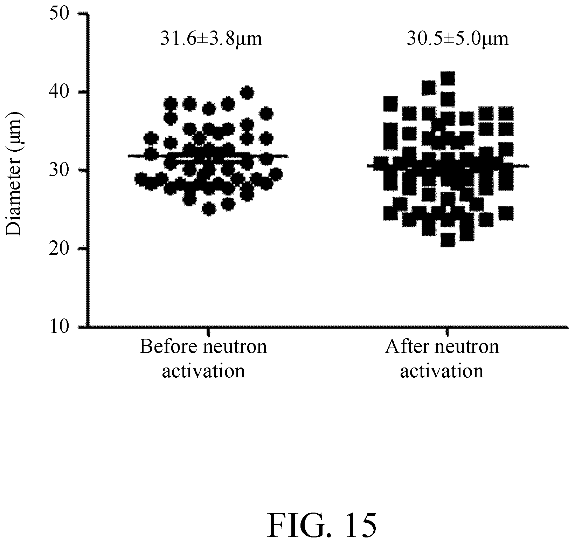

In the Example, the glass microspheres were observed for the changes in size and appearance before and after neutron activation. The yttrium-90/copper-64 dual nuclides glass microspheres before neutron activation and the yttrium-90/copper-64 dual nuclides glass microspheres after neutron activation and with the activity decayed to the environmental background (completely decayed) were placed on a glass slide, and sample images were captured using a upright fluorescence microscope (Olympus BX61) at magnifications of 10X and 40X, as shown in FIG. 14. The diameter of each microsphere in the images was calculated through the MetaMorph® image processing software, and the data was imported into the GraphPad Prism software for analysis and plotting the microsphere diameter distribution chart, as shown in FIG. 15.

According to FIG. 14, the glass microspheres had an appearance of smooth spherical shape both before and after neutron activation, indicating that the neutron activation could not alter the appearance of the glass microspheres significantly. Additionally, according to FIG. 15, the glass microspheres had a diameter of 31.6 ± 3.8 μm and 30.5 ± 0.5 μm before and after neutron activation, respectively, indicating that the diameter distribution had no significant difference before and after neutron activation.

Example 12

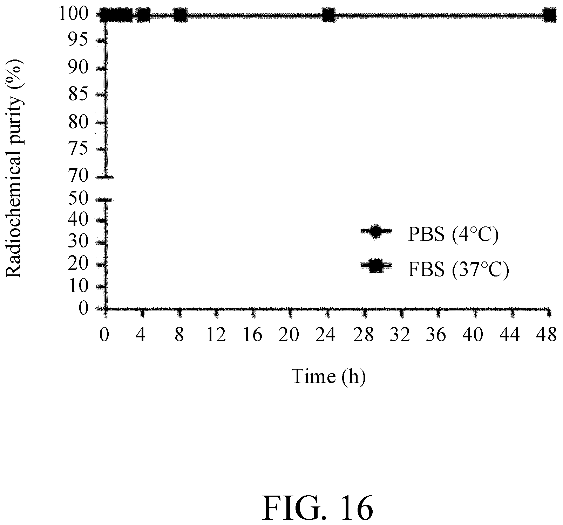

In vitro stability analysis was performed on the yttrium-90/copper-64 dual nuclides glass microspheres. 24 Eppendorf tubes were charged with the glass microspheres with fixed activity, respectively, and were also charged phosphate buffer solution (PBS) or fetal bovine serum (FBS). The Eppendorf tubes were placed in incubators at 4°C or 37°C, respectively, and 3 tubes were taken out at every selected time point (1, 2, 4, 8, 24 and 48 hours) for radiochemical purity assay. 200 μL sample was filtered through 0.45 μm filter, then the filter was rinsed with 2 mL physiological saline, and the filtrate was collected. The residual activity of the filtrate and the filter were measured, respectively, the radiochemical purity was calculated according to the following equation, and the time-radiochemical purity relationship chart was plotted (FIG. 16):

Radiochemical purity (%) = [Residual activity of filter/(Residual activity of filter + filtrate)] ×100%

According to FIG. 16, the yttrium-90/copper-64 dual nuclides glass microspheres had a radiochemical purity still maintained at >98% after they were left to stand for 48 hours in PBS/4°C and in FBS/37°C, indicating that the yttrium-90/copper-64 dual nuclides glass microspheres had very good stability. It could be concluded that the glass microspheres were not easy to be chelated by the proteins in blood after administration to a body.

Example 13

In vivo stability analysis was performed on the yttrium-90/copper-64 dual nuclides glass microspheres. Sprague-Dawley (SD) rats of 8-10 weeks old were gaseously anesthetized with isoflurane/oxygen (2%) and then were fixed on a platform. 300 μL of liquid containing yttrium-90/copper-64 glass microspheres (in which the weight of glass microspheres was about 500 μCi/50 mg) was injected via caudal vein. PET/CT imaging was performed at each selected time point (1, 4, 24 and 48 hours), the left and right lung lobes were picked as the ROIs, and a quantitative analysis was performed by using an image analysis software to calculate the photon signal intensities in the ROIs.

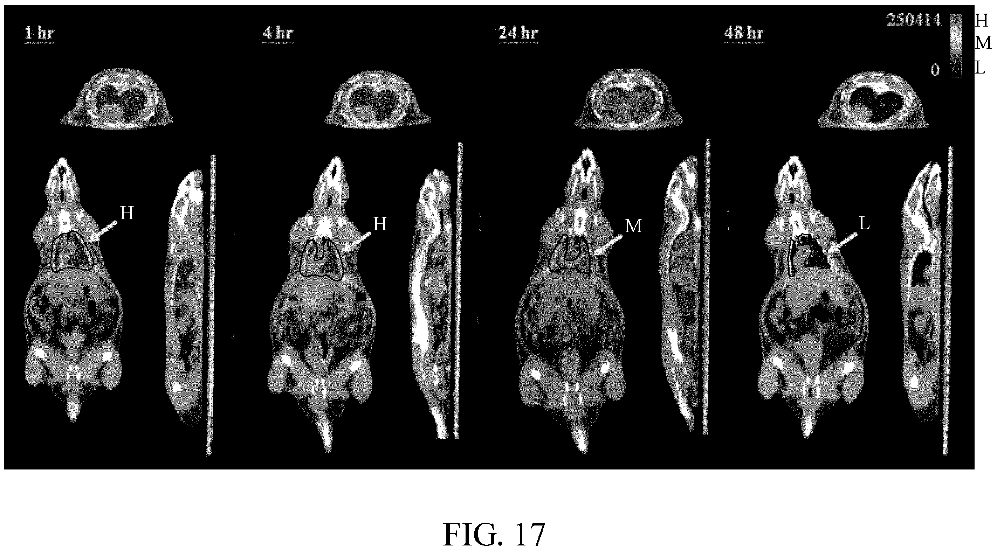

The PET/CT imaging results in Fig. 17 showed that the glass microspheres were almost completely occluded in the pulmonary microvasculature within 1 hour after intravenous injection, resulting in high activity in the lungs (left and right lobes) (the lung area was briefly labeled as the high activity (H) area); but in other parts of the body, except for some residual glass microspheres in the tail vein at the injection site, there was almost no accumulation, and the activity is very low, approaching zero. 4 hours after injection, the activity of glass microspheres in the lungs and the injection site at the tail vein only slightly decreased and remained at a high activity level. Over time, the activity of glass microspheres in the lungs and the injection site at the tail vein decreased 24 hours after injection (for simplicity, the lung region was identified as the medium activity (M) region). 48 hours after injection, the glass microspheres in the lungs and the injection site at the tail vein showed a more significant decrease (the lung area was identified as a low activity (L) area), with activity almost the same as in other areas of the body where glass microspheres did not accumulate.

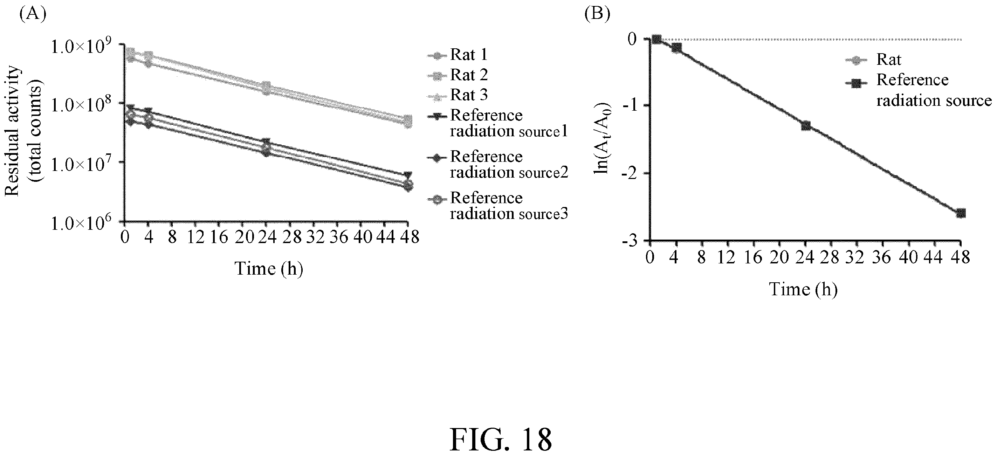

FIG. 18 (A) and (B) showed the time-residual activity At chart and time-ln($A_t/A_0$) chart of the left and right lung lobes of rats and the reference radiation source picked as the ROIs, respectively. According to the calculation mode described in Example 8, the effective half-lives of yttrium-90 and copper-64 contained in glass microspheres in the left and right lung lobes of rats in vivo were 12.48 hours and 53.23 hours, respectively. Additionally, from the group where the reference radiation source was picked as the ROI, the physical half-lives of yttrium-90 and copper-64 were 12.48 and 67.35 hours, respectively, which were close to the known half-lives.

Further, the physiological half-life of the yttrium-90/copper-64 dual nuclides glass microspheres calculated according to Equation 6 below was also very long,

$$\frac{1}{T_E} = \frac{1}{T_R} + \frac{1}{T_B} \quad \text{[Equation 6]}$$

wherein $T_E$ is effective half-life, $T_R$ is physical half-life, and $T_B$ is physiological half-life.

Based on the in vitro and in vivo stability results of Examples 12 and 13, in the glass microspheres of the present disclosure, the yttrium-90 and copper-64 used as radioactive nuclides did not leak during the experimental period and could persist in the glass microspheres. The glass microspheres persistently occluded in lungs after intravenous administration, which could be considered to have a permanent embolization, thus the microspheres can be used in a specific internal radiation therapy.

**Claims**

1.    A microsphere, comprising:

a plurality of pores having a gradient characteristic gradually decreasing along a direction from a surface to a center of the microsphere;
a first nuclide distributed in the microsphere and having a concentration in the center of the microsphere higher than that in the surface of the microsphere; and
a second nuclide distributed in the microsphere and a distribution of the second nuclide decreases gradually along the direction from the surface to the center of the microsphere,
wherein the first nuclide and the second nuclide after neutron activation are radioactive and produce β-rays, γ-rays or a combination thereof.

2.    The microsphere of claim 1, wherein the gradient characteristic comprises diameters of the pores, the distribution of the pores, or a combination thereof.

3.    The microsphere of claim 1, wherein the pores are free from penetrating the microsphere.

4.    The microsphere of claim 1, wherein the plurality of pores comprise macropores and micropores, wherein the macropores have diameters of 10-30 microns, and the micropores have diameters of 0.1-10 microns.

5.    The microsphere of claim 1, wherein the first nuclide is selected from yttrium, aluminum, silicon, or a combination thereof.

6.    The microsphere of claim 1, wherein the second nuclide comprises at least one selected from the group consisting of: potassium, barium, molybdenum, tellurium, indium, antimony, gallium, zinc, zirconium, palladium, rhodium, tantalum, tungsten, iridium, platinum, niobium, technetium, strontium, titanium, vanadium, phosphorus, calcium, sodium, rhenium, scandium, lanthanum, cerium, praseodymium, neodymium, promethium, samarium, europium, gadolinium, terbium, dysprosium, holmium, erbium, thulium, ytterbium, lutetium, copper, gold, silver, iron, tin, cobalt, nickel, manganese, aluminum, carbon, boron, iodine, actinium-225, antimony-127, arsenic-74, barium-140, bismuth-210, bismuth-213, californium-246, calcium-46, calcium-47, carbon-11, carbon-14, cesium-131, cesium-137, chromium-51, cobalt-57, cobalt-58, cobalt-60, dysprosium-165, dysprosium-166, erbium-169, erbium-109, fluorine-18, gallium-67, gallium-68, gold-198, holmium-166, hydrogen-3, indium-111, indium-113m, iodine-123, iodine-125, iodine-131, iridium-192, iridium-194, iron-59, iron-82, krypton-81m, lanthanum-140, lutetium-177, molybdenum-99, nitrogen-13, oxygen-15, palladium-103, phosphorus-32, radon-222, radium-224, radium-223, rhenium-186, rhenium-188, rhodium-82, samarium-153, selenium-75, sodium-22, sodium-24, strontium-89, strontium-90, technetium-99m, thallium-201, xenon-127, xenon-133, cerium-137, actinium-225, zirconium-89, terbium-149, astatine-211, thorium-227, thorium-201, bismuth-212, bismuth-213, copper-64, ytterbium-169, ytterbium-175, lead-212, potassium-42, rubidium-82, titanium-45, scandium-44, and yttrium-90.

7. The microsphere of claim 1, further comprising a shell layer that is coated on the surface of the microsphere and filled into the plurality of pores, wherein the shell layer comprises a material selected from organic materials, inorganic materials, or a combination thereof, preferably the organic materials are selected from polyvinylpyrolidone, polyvinyl alcohol, carboxymethylcellulose, methylcellulose, hydroxypropylmethylcellulose, hydroxypropylcellulose, polyethylene glycol, Arabic gum, polylactic acid, polylactic acid-glycolic acid, or a combination thereof, preferably the inorganic materials are selected from phosphate, sulfate, chloride, nitrate, telluride, tellurate, iodide, iodate, xenate, tungstate, rhenate platinate, aurichloride, mercurate, plumbate, bismuthate, astatate, uranate, polonide, osmate, antimonate, stannate, stannide, technetate, molybdate, niobate, bromate, bromide, selenate, selenide, arsenate, zincate, cuprate, cobaltate, ferrate, nickelate, manganate, chromate, vanadate, titanate, chlorate, sulfide, fluorophosphate, fluorosilicate, silicate, aluminate, fluoride, oxide, peroxide, superoxide, cyanate, carbonate, or borate.

8. The microsphere of claim 1, which has a diameter of 2-1000 microns.

9. The microsphere of claim 1, wherein the first nuclide has an etching resistance higher than that of the second nuclide.

10. A method for preparing a microsphere, comprising:

providing a first raw material of a first nuclide and a second raw material of a second nuclide, wherein the first raw material and the second raw material are powders;
mixing the first raw material and the second raw material to form a mixed powder;
heating the mixed powder to obtain an intermediate;
melting and spherizing the intermediate to form a molten droplet;
contacting the molten droplet with a cooling medium to obtain a microsphere, wherein the first nuclide is distributed in the microsphere and has a concentration in the center of the microsphere higher than that in the surface of the microsphere, and the second nuclide has a distribution decreasing gradually along a direction from the surface to the center of the microsphere; and
contacting the microsphere with a treatment solution to form a plurality of pores, wherein the plurality of pores have a gradient characteristic decreasing gradually along the direction from the surface to the center of the microsphere,
wherein the first nuclide and the second nuclide after neutron activation are radioactive and produce $\beta$-rays, $\gamma$-rays or a combination thereof.

11. A method for preparing a microsphere, comprising:

providing a first raw material of a first nuclide, wherein the first raw material is a powder;
heating the first raw material to obtain an intermediate;
melting and spherizing the intermediate to form a molten droplet;
contacting the molten droplet with a cooling medium to obtain a microsphere, wherein the cooling medium is a second raw material of a second nuclide, and the second raw material is a liquid, allowing the second nuclide to enter the molten droplet or the microsphere from the surface and to be distributed therein or precipitate on the surface, resulting in that the first nuclide is distributed in the microsphere and has a concentration in the center of the microsphere higher than that in the surface of the microsphere, and the second nuclide has a distribution decreasing gradually along a direction from the surface to the center of the microsphere; and
contacting the microsphere with a treatment solution to form a plurality of pores, wherein the plurality of pores have a gradient characteristic decreasing gradually along the direction from the surface to the center of the microsphere,
wherein the first nuclide and the second nuclide after neutron activation are radioactive and produce $\beta$-rays, $\gamma$-rays or a combination thereof.

12. The method of claim 10, wherein the cooling medium is a second raw material of a second nuclide, and the second raw material is a liquid, allowing the second nuclide to enter the molten droplet or the microsphere from the surface and to be distributed therein or precipitate on the surface.

13. The method of any one of claims 10-11, wherein the treatment solution comprises an etching agent.

14. The method of claim 13, wherein the etching agent is a combination of one of an acid and a base with an oxidant, the acid is at least one selected from the group consisting of citric acid, lactic acid, oxalic acid, acetic acid, permanganic acid, p-toluenesulfonic acid, phosphoric acid, aqua regia, hydrofluoric acid, hydrochloric acid, hydrobromic acid, hydroiodic acid, nitric acid, sulfuric acid, perchloric acid, chloric acid ($HClO_3$), bromic acid ($HBrO_3$), perbomic acid ($HBrO_4$), iodic acid ($HIO_3$), periodic acid ($HIO_4$), metaperiodic acid ($HIO_4$), selenic acid ($H_2SeO_4$), fluorosilicic acid ($H_2SiF_6$), chlorioplumbic acid ($H_2PbCl_6$), ferric acid ($H_2FeO_4$), fluoroboric acid ($HBF_4$), fluorosulfonic acid ($HSO_3F$), cyanic acid (HOCN), thiocyanic acid (HSCN), 2,4,6-trinitrophenol ($HC_6H_2N_3O_7$), 2,4,6-trinitrobenzoic acid ($HC_7H_2N_3O_8$), trifluoroacetic acid ($CF_3COOH$), trichloroacetic acid ($CCl_3COOH$), methanesulfonic acid ($CH_3SO_3H$), benzenesulfonic acid ($C_6H_5SO_3H$), mercaptocyclohexanesulfonic acid ($C_6H_{10}(SH)SO_3H$), 2-chloroethane thiol ($CH_3CHClSH$), fluoroantimonic acid ($HSbF_6$), magaic acid ($SbF_6SO_3H$), perfluorinated sulfonic acid resin (Nafion-H), chlorofluoroaluminic acid ($HAlCl_3F$), carbonboronoic acid ($H[CHB_{11}Cl_{11}]$), and $FeCl_3 \cdot HClO_4 \cdot SiO_2 \cdot nH_2O$; the base is at least one selected from the group consisting of sodium hydroxide, potassium hydroxide, calcium hydroxide, ammonia, lithium hydroxide, rubidium hydroxide, cesium hydroxide, francium hydroxide, strontium hydroxide, barium hydroxide, radium hydroxide, thallous hydroxide, silver diamminohydroxide, choline, quaternary ammonium base, butyl lithium, lithium diisopropylamide, benzyl lithium, Grignard reagent, alkyl copper lithium, sodium methoxide, sodium ethoxide, potassium etyoxide, and sodium tert-butoxide; and the oxidant is at least one selected from the group consisting of an amphoteric compound, hydrogen peroxide, permanganate, hypochlorite, chromate, dichromate, and chromium trioxide.

15. The method of any one of claims 10-11, wherein the treatment solution performs etching at a position of the second nuclide.

EP 4 578 496 A1

FIG. 1A

EP 4 578 496 A1

FIG. 1B

**FIG. 1C**

FIG. 2

FIG. 3

(A)

(B)

(C)

Contacting with 1M CuSO$_4$
5H$_2$O solution

Contacting with 3M CuSO$_4$
5H$_2$O solution

Contacting with 10M CuSO$_4$
5H$_2$O solution

FIG. 4

FIG. 5

FIG. 6

EP 4 578 496 A1

(A)　(B)

FIG. 7

FIG. 8

FIG. 9

FIG. 10

FIG. 11

(A) In Eppendorf tube

$\ln(A_t/A_0)$ vs Time (h)

- 201.5mg
- 100mg
- 50.7mg
- 24.5mg
- 11.1mg
- 6.0mg

(B) In agar

$\ln(A_t/A_0)$ vs Time (h)

- 201.5mg
- 100mg
- 50.7mg
- 24.5mg
- 11.1mg
- 6.0mg

FIG. 12

FIG. 13

FIG. 14

FIG. 15

FIG. 16

FIG. 17

EP 4 578 496 A1

FIG. 18

Europäisches
Patentamt
European
Patent Office
Office européen
des brevets

# EUROPEAN SEARCH REPORT

**Application Number**

EP 24 21 7498

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/205584 A1 (SIMMONS BRANDON DAVID [US]) 8 July 2021 (2021-07-08) * paragraphs [0068] - [0085] * | 1-15 | INV. A61N5/10 G21G4/06 |
| A | WO 2004/040972 A2 (BIOSPHERE MEDICAL [US]) 21 May 2004 (2004-05-21) * page 5, line 26 - page 6, line 11 * | 1-15 | |
| A | US 2011/212179 A1 (LIU DAVID [US]) 1 September 2011 (2011-09-01) * paragraphs [0002], [0055] - [0076] * * figure 1 * | 1-15 | |
| A | US 2003/183962 A1 (BUISER MARCIA [US] ET AL) 2 October 2003 (2003-10-02) * paragraphs [0009], [0030] * | 1-15 | |

**TECHNICAL FIELDS
SEARCHED      (IPC)**

A61N
G21G

The present search report has been drawn up for all claims

| Place of search | Date of completion of the search | Examiner |
|---|---|---|
| Munich | 27 April 2025 | Kretschmann, Jana |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 24 21 7498

This annex lists the patent family members relating to the patent documents cited in the above-mentioned European search report.
The members are as contained in the European Patent Office EDP file on
The European Patent Office is in no way liable for these particulars which are merely given for the purpose of information.

27-04-2025

| Patent document cited in search report | Publication date | Patent family member(s) | Publication date |
|---|---|---|---|
| US 2021205584 A1 | 08-07-2021 | AU 2019269701 A1 | 14-01-2021 |
| | | AU 2019269705 A1 | 07-01-2021 |
| | | CN 112384988 A | 19-02-2021 |
| | | CN 112423822 A | 26-02-2021 |
| | | CN 118356553 A | 19-07-2024 |
| | | CN 118356554 A | 19-07-2024 |
| | | CN 118512685 A | 20-08-2024 |
| | | CN 118662732 A | 20-09-2024 |
| | | EP 3793659 A1 | 24-03-2021 |
| | | EP 3794608 A1 | 24-03-2021 |
| | | IL 278804 A | 31-01-2021 |
| | | IL 278811 A | 31-01-2021 |
| | | JP 7361724 B2 | 16-10-2023 |
| | | JP 7482789 B2 | 14-05-2024 |
| | | JP 7584573 B2 | 15-11-2024 |
| | | JP 2021524307 A | 13-09-2021 |
| | | JP 2021524308 A | 13-09-2021 |
| | | JP 2023113944 A | 16-08-2023 |
| | | JP 2024161511 A | 19-11-2024 |
| | | NZ 770696 A | 31-05-2024 |
| | | NZ 770991 A | 31-05-2024 |
| | | US 2021205584 A1 | 08-07-2021 |
| | | US 2021213301 A1 | 15-07-2021 |
| | | US 2024325692 A1 | 03-10-2024 |
| | | WO 2019222687 A1 | 21-11-2019 |
| WO 2004040972 A2 | 21-05-2004 | AU 2003285905 A1 | 07-06-2004 |
| | | CA 2507404 A1 | 21-05-2004 |
| | | EP 1578455 A2 | 28-09-2005 |
| | | WO 2004040972 A2 | 21-05-2004 |
| US 2011212179 A1 | 01-09-2011 | CN 102143996 A | 03-08-2011 |
| | | EP 2344578 A2 | 20-07-2011 |
| | | JP 2012507562 A | 29-03-2012 |
| | | US 2011212179 A1 | 01-09-2011 |
| | | WO 2010062678 A2 | 03-06-2010 |
| US 2003183962 A1 | 02-10-2003 | AU 2003222097 A1 | 13-10-2003 |
| | | CA 2480631 A1 | 09-10-2003 |
| | | DE 60308159 T2 | 09-08-2007 |
| | | EP 1490032 A1 | 29-12-2004 |
| | | JP 4533631 B2 | 01-09-2010 |
| | | JP 2005529193 A | 29-09-2005 |
| | | US 2003183962 A1 | 02-10-2003 |
| | | US 2003185896 A1 | 02-10-2003 |
| | | US 2006210710 A1 | 21-09-2006 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 21 7498

This annex lists the patent family members relating to the patent documents cited in the above-mentioned European search report.
The members are as contained in the European Patent Office EDP file on
The European Patent Office is in no way liable for these particulars which are merely given for the purpose of information.

27-04-2025

| Patent document cited in search report | Publication date | Patent family member(s) | | Publication date |
|---|---|---|---|---|
| | | WO | 03082250 A1 | 09-10-2003 |

EPO FORM P0459

For more details about this annex : see Official Journal of the European Patent Office, No. 12/82

page 2 of 2